# EUROPEAN PATENT SPECIFICATION

(11) **EP 1 737 487 B1**
(45) Date of publication and mention of the grant of the patent: **25.05.2016**
(21) Application number: 05732213.3
(22) Date of filing: 30.03.2005
(51) Int. Cl.: A61K 39/395, A61P 37/00, A61K 39/00

(54) **METHOD FOR TREATING AUTOIMMUNE DISEASES WITH ANTIBODIES**
VERFAHREN ZUR BEHANDLUNG VON AUTOIMMUNKRANKHEITEN MIT ANTIKÖRPERN
METHODE SERVANT A TRAITER DES MALADIES AUTO-IMMUNES AU MOYEN D'ANTICORPS

(30) Priority: 30.03.2004 US 558080 P; 29.09.2004 US 613712 P
(43) Date of publication of application: 03.01.2007
(73) Proprietor: Canadian Blood Services, Ottawa, Ontario K1G 4J5 (CA)
(72) Inventor: LAZARUS, Alan H., Toronto, Ontario M4H 1J6 (CA); SIRAGAM, Vinayakumar, Toronto, Ontario M6J 3N8 (CA); BRINC, Davor, Toronto, Ontario M4Y 2V6 (CA); FREEDMAN, John, Toronto, Ontario M4Y 1R8 (CA); SONG, Seng, Toronto, Ontario M2H 1X2 (CA); CROW, Andrew R., Scarborough, ON M1H 2P4 (CA)
(74) Representative: Engelhard, Markus
(86) International application number: PCT/CA2005/000472
(87) International publication number: WO 2005/094880

(56) References cited:
- WO-A1-99/03495
- WO-A2-02/40047
- WO-A2-02/076384
- CA-A1- 2 253 058
- US-A1- 2003 059 937
- SONG S ET AL: "Monoclonal IgG can ameliorate immune thrombocytopenia in a murine model of ITP: An alternative to IVIG" BLOOD, W.B.SAUNDERS COMPANY, ORLANDO, FL, US, vol. 101, no. 9, May 2003 (2003-05), pages 3708-3713, XP002374394 ISSN: 0006-4971
- LAZARUS A H ET AL: "Monoclonal antibodies which mimic the action of intravenous immunoglobulin (mIVIG) can inhibit immune thrombocytopenia" BIOSIS, 2001, XP002374393
- BUSSEL J B: "EC RECEPTOR BLOCKADE AND IMMUNE THROMBOCYTOPENIC PURPURA" SEMINARS IN HEMATOLOGY, PHILADELPHIA, PA, US, vol. 37, no. 3, July 2000 (2000-07), pages 261-266, XP008011545 ISSN: 0037-1963
- BUSSEL J B: "The use of intravenous gamma-globulin in idiopathic thrombocytopenic purpura" MEDLINE, 1989, XP002226230
- ERICSON S G ET AL: "MONOCLONAL ANTIBODY 197 (ANTI-FCGAMMARI) INFUSION IN A PATIENT WITHIMMUNE THROMBOCYTOPENIA PURPURA (ITP) RESULTS IN DOWN-MODULATION OF FCGAMMARI ON CIRCULATING MONOCYTES" BRITISH JOURNAL OF HAEMATOLOGY, OXFORD, GB, vol. 92, no. 3, March 1996 (1996-03), pages 718-724, XP000199685 ISSN: 0007-1048
- SONG S. ET AL: 'Monoclonal IgG can ameliorate immune thrombocytopenia in a murine model of ITP: an alternative to IVIG.' BLOOD. vol. 101, no. 9, 01 May 2003, pages 3708 - 3713, XP002374394
- SIRAGAM M. ET AL: 'Can antibodie with specificity for soluble antigens mimic the therapeutic effects to intravenous IgG in the treatmen of autoimmune disease?' J CLIN INVEST. vol. 115, no. 1, January 2005, pages 155 - 160, XP008092777
- KUTER D. ET AL: 'The reciprocal relationship of thrombopoietin (c-Mpl ligand) to chenges in the patelet mass during busulfan-induced thrombocytopenia in the rabbit.' BLOOD. vol. 85, no. 10, 15 May 1995, pages 2720 - 2730, XP008090427
- AIGER S. ET AL: 'CD24 mediates rolling of breast carcinoma cells on P-selectin.' FASEB J. vol. 12, no. 12, August 1998, pages 1241 - 1251, XP001190894
- RISTAMAKI R. ET AL: 'Serum CD44 in malignant lymphoma: an association with treatment response.' BLOOD. vol. 84, no. 1, 01 July 1994, pages 238 - 243, XP008090428
- KENNETH G. C. SMITH ET AL: 'Fc[gamma]RIIB in autoimmunity and infection: evolutionary and therapeutic implications' NATURE REVIEWS IMMUNOLOGY vol. 10, no. 5, 01 May 2010, pages 328 - 343, XP055000315 DOI: 10.1038/nri2762 ISSN: 1474-1733
- SIRAGAM: 'Can antibodies with specificity for soluble antigens mimic the therapeutic effects of intravenous IgG in the treatment of autoimmune disease?', [Online] 01 January 2005, pages 155 - 160, XP055000330 Retrieved from the Internet: <URL:http://www.ncbi.nlm.nih.gov/pmc/articl es/PMC539200/pdf/JCI0422753.pdf> [retrieved on 2011-06-08]
- ALAN LAZARUS: 'Intravenous immunoglobulin and Anti-D in Idiopathic Thrombocytopenic Purpura (ITP): Mechanisms of Action' TRANSFUS. SCI., [Online] 01 January 1998, pages 289 - 294, XP055000369 Retrieved from the Internet: <URL:http://www.sciencedirect.com/science?_ ob=MImg&_imagekey=B6TCT-3V7RG00-N-3&_cdi=51 79&_user=987766&_pii=S0955388698000435&_ori gin=&_coverDate=09%2F30%2F1998&_sk=99980999 6&view=c&wchp=dGLzVzb-zSkWz&md5=eaea69e4e2f 96ed58f6c1781cdbf57cb&ie=/sdarticle.pdf> [retrieved on 2011-06-09]
- N WEBBER: 'Functional properties of lymphocytes in idiopathic thrombocytopenic purpura' HUMAN IMMUNOLOGY vol. 62, no. 12, 01 December 2001, pages 1346 - 1355, XP055000411 DOI: 10.1016/S0198-8859(01)00348-2 ISSN: 0198-8859
- ELLIOTT ET AL: "2 Anti-cytokine therapy in rheumatoid arthritis", BAILLIERE'S BEST PRACTICE AND RESEARCH. CLINICAL REUMATOLOGY, BAILLIERE TINDALL, LONDON, GB, vol. 9, no. 4, 1 November 1995 (1995-11-01), pages 633-652, XP005814700, ISSN: 1521-6942, DOI: DOI:10.1016/S1521-6942(05)80013-0
- LAZARUS ALAN H ET AL: "Mechanism of action of IVIG and anti-D in ITP", TRANSFUSION AND APHERESIS SCIENCE, ELSEVIER SCIENCE, LONDON, GB, vol. 28, no. 3, 1 June 2003 (2003-06-01), pages 249-255, XP002464550, ISSN: 1473-0502, DOI: DOI:10.1016/S1473-0502(03)00043-0
- LUBICA RAUOVA ET AL: "Immunomodulation of autoimmune diseases by high-dose intravenous immunoglobulins", SPRINGER SEMINARS IN IMMUNOPATHOLOGY, SPRINGER, BERLIN, DE, vol. 23, no. 4, 1 December 2001 (2001-12-01), pages 447-457, XP019708248, ISSN: 1432-2196
- VASSILEV T L ET AL: "Variable Region-Connected, Dimeric Fraction of Intravenous immunoglobulin Enriched in Natural Autoantibodies", JOURNAL OF AUTOIMMUNITY, LONDON, GB, vol. 8, no. 3, 1 January 1995 (1995-01-01) , pages 405-413, XP002265834, ISSN: 0896-8411, DOI: DOI:10.1006/JAUT.1995.0032
- RAUOVA L ET AL: "Immunomodulation of autoimmune diseases by high-dose intravenous immunoglobulins.", SPRINGER SEMINARS IN IMMUNOPATHOLOGY DEC 2001 LNKD- PUBMED:11826620, vol. 23, no. 4, December 2001 (2001-12), pages 447-457, ISSN: 0344-4325
- KAZATCHKINE M D ET AL: "Immunomodulation of autoimmune and inflammatory diseases with intravenous immune globulin.", THE NEW ENGLAND JOURNAL OF MEDICINE 6 SEP 2001 LNKD- PUBMED:11547745, vol. 345, no. 10, 6 September 2001 (2001-09-06), pages 747-755, ISSN: 0028-4793
- YI WANG: 'Anti-C5 monoclonal antibody therapy prevents collagen-induced arthritis and ameliorates established disease', [Online] 01 September 1995, pages 8955 - 8959, XP055000476 Retrieved from the Internet: <URL:http://www.pnas.org/content/92/19/8955 .full.pdf> [retrieved on 2011-06-10]
- ANONYMOUS: 'Immune Globulin (Human)', [Online] 01 January 2003, XP055114738 Retrieved from the Internet: <URL:https://www.seacoastmedical.com/storef rontCommerce/forms/ProductInfo/BayGam.pdf> [retrieved on 2014-04-22]
- SAWYER LEIGH A: "Antibodies for the prevention and treatment of viral diseases", ANTIVIRAL RESEARCH, ELSEVIER BV, NL, vol. 47, no. 2, 1 August 2000 (2000-08-01) , pages 57-77, XP002302232, ISSN: 0166-3542, DOI: 10.1016/S0166-3542(00)00111-X
- H U SIMON ET AL: 'IVIG - mechanisms of action' ALLERGY, [Online] 01 January 2003, pages 543 - 552, XP055114974 Retrieved from the Internet: <URL:http://onlinelibrary.wiley.com/store/1 0.1034/j.1398-9995.2003.00239.x/asset/j.139 8-9995.2003.00239.x.pdf?v=1&t=huco8cxi&s=1b 145b902324f7b10e9a25240954289d892f27f8> [retrieved on 2014-04-23]
- P L MASSON: 'Elimination of Infectious Antigens and Increase of IgG Catabolism as Possible Modes of Action of IVIg' JOURNAL OF AUTOIMMUNITY, [Online] 01 January 1993, pages 683 - 689, XP055114975 Retrieved from the Internet: <URL:http://www.sciencedirect.com/science/a rticle/pii/S0896841183710577/pdf?md5=b70ac4 5ba7a7f7c60e81f85783af0356&pid=1-s2.0-S0896 841183710577-main.pdf> [retrieved on 2014-04-23]
- J. M.M. Den Haan ET AL: "Constitutive versus Activation-dependent Cross-Presentation of Immune Complexes by CD8+ and CD8- Dendritic Cells In Vivo", Journal of Experimental Medicine, vol. 196, no. 6, 16 September 2002 (2002-09-16), pages 817-827, XP055201943, ISSN: 0022-1007, DOI: 10.1084/jem.20020295
- R Consden ET AL: "Production of a chronic arthritis with ovalbumin. Its retention in the rabbit knee joint.", Annals of the rheumatic diseases, vol. 30, no. 3, 1 May 1971 (1971-05-01), pages 307-315, XP055202078, ISSN: 0003-4967, DOI: 10.1136/ard.30.3.307
- KOJIMA T ET AL: "Early degradation of type IX and type II collagen with the onset of experimental inflammatory arthritis", ARTHRITIS & RHEUMATISM, WILEY, US, vol. 44, no. 1, 1 January 2001 (2001-01-01), pages 120-127, XP003023406, ISSN: 0004-3591, DOI: 10.1002/1529-0131(200101)44:1<120::AID-ANR 16>3.0.CO;2-X

## Description

### TECHNICAL FIELD

This application relates to the treatment of autoimmune diseases using antibodies. More preferably, the present invention relates to treatment of autoimmune diseases with soluble antigen-specific antibodies.

### BACKGROUND OF THE INVENTION

Immune thrombocytopenic purpura (ITP) is an autoimmune disease characterised by platelet clearance mediated by pathogenic anti-platelet antibodies. It has been previously suggested that this platelet clearance is mediated by Fcγ receptor (FcγR)-bearing macrophages in the reticuloendothelial system (RES). While intravenous immunoglobulin (IVIg) is widely used in the treatment of ITP as well as in a wide variety of chronic autoimmune and inflammatory diseases, its mechanism of action is not yet fully elucidated. Possible mechanisms of action include inhibition of RES function, anti-idiotype antibodies and immunomodulation. In murine models of ITP, it has been demonstrated that IVIg ameliorates ITP by a mechanism completely dependent upon the expression of the inhibitory FcγRIIB. In humans, there is also evidence that IVIg increases the level of expression of FcγRIIB. In addition, it has been previously reported that the clinical effects of IVIg as well as monoclonal mimetics of IVIg both ameliorate murine ITP in a manner that correlates with RES blockade. This 'competitive' RES blockade has long been considered to be the primary mechanism whereby IVIg ameliorates ITP.

The present study was undertaken to investigate if antibodies to soluble antigens could inhibit autoimmune diseases.

### SUMMARY OF THE INVENTION

According to the present invention, a novel pharmaceutical composition for use in treating ITP is provided in accordance with the appended claims. More specifically, the present invention relates to a pharmaceutical composition for use in treating an immune thrombocytopenia in a mammal characterized as comprising (i) antibody-antigen complexes obtained by incubating at least one IgG antibody specific for ovalbumin together with ovalbumin and (ii) a pharmaceutical acceptable carrier. It also relates to the use of at least one IgG antibody specific for ovalbumin for the manufacture of a medicament for the treatment of an immune thrombocytopenia, characterized in that ovalbumin and said IgG antibody are incubated together prior to the administration to said mammal to form antibody-antigen or antibody-antigen-blood cell complexes for manufacturing the medicament. In one embodiment, the use of at least one IgG antibody specific for ovalbumin for the manufacture of a medicament for the treatment of an immune thrombocytopenia, is further characterized in that the therapeutic amount of said at least one IgG antibody is from 0.1 µg to 1 g per kg of body weight per day. Furthermore, a novel mechanism of action has been established in accordance with the present invention for antibody-based treatment regimes for ITP, including, but not limited to anti-CD44 and soluble antigen specific antibody treatment regimes.

Also disclosed, but not encompassed by the present invention is a method for treating autoimmune diseases in a mammal which method comprises administering to the mammal an effective amount of at least one antibody specific for a soluble antigen.

According to the present invention, the autoimmune disease is Immune thrombocytopenia that is treated using the pharmaceutical composition of the present invention.

As disclosed in the present application,, the treatment can be effected for a time and under conditions sufficient to inhibit platelet clearance, thereby treating or ameliorating immune thrombocytopenic purpura (ITP). It is also disclosed, but does not form part of the present invention, that inflammatory arthritis can be prevented or ameliorated by the administration of antibodies to a soluble antigen.

The soluble antigen can either be an endogenous or a foreign antigen. By foreign antigen it is meant an antigen that is not normally produced by the same individual or species. The antigen can be a non-functional/inert antigen. In another embodiment the binding of the antibody to the antigen does not compromise the function of the antigen.

In an aspect of the disclosure the soluble foreign antigen and the antibody can be incubated together to form antibody-antigen complexes prior to administering the complexes to the mammal.

In another aspect of the disclosure the endogenous soluble antigen can be obtained from the mammal and incubated with the antibody to form antibody-antigen complexes, the complexes being subsequently administered to the mammal. Alternatively, a soluble antigen may be injected into a mammal having a pre-existing antibody of interest specific to the soluble antigen, e.g. a mammal who has been previously immunised to tetanus toxin (any # of years earlier) may be administered an injection of soluble tetanus toxin according to an alternate embodiment of the present invention.

The antibody can be administered intravenously, interperitoneally, intradermally, intramuscularly, subcutaneously, orally or rectally.

In another embodiment of the disclosure the soluble antigen can be associated with blood cells and the resulting antigen-cell complexes can be targeted by antibodies for inhibiting platelet clearance and thereby treating thrombocytopenia.

It is also disclosed that autoimmune disease treatment regime is provided to mediate a cellular response in dendritic cells, such as leukocytes, such that platelet clearance is slowed and/or inhibited, thereby treating or ameliorating an autoimmune disease.
According to the invention there are provided pharmaceutical compositions for treating thrombocytopenia, comprising antibody-antigen complexes obtained by incubating at least one IgG antibody specific for ovalbumin together with ovalbumin and a pharmaceutical acceptable carrier. In yet another aspect of the invention, at least one IgG antibody specific for ovalbumin may be used in the manufacture of a medicament for the treatment of ITP.

By the present invention, we demonstrate that antibodies to soluble antigens can ameliorate ITP in an FcγRIIB-dependent manner. Antibody directed to the cell-associated antigen inhibited ITP in an FcγRIIB-independent manner. Taken together, these data demonstrate that IgG antibodies reactive with either a soluble or insoluble antigen can mimic the effects of IVIg. In addition, the mechanisms of action of these moieties are quite different: antibody reacted with soluble antigen may utilize the same pathway used by IVIg, i.e. an FcγRIIB-dependent pathway, whereas antibody reacted with a cell-associated antigen may work by additional and/or other mechanisms of action, and possibly by competitive RES inhibition.

### BRIEF DESCRIPTION OF THE DRAWINGS

Further features and advantages of the present invention will become apparent from the following detailed description, taken in combination with the appended drawings, in which:
Figs. 1A and 1B illustrate the association of OVA on the surface of RBCs.
Figs. 2A and 2B illustrates inhibition of thrombocytopenia by treating OVA-coupled RBCs with OVA-specific IgG.
Figs. 3A, 3B and 3C illustrates amelioration of thrombocytopenia with antibodies reactive with soluble OVA (in combination with soluble OVA) ameliorate immune thrombocytopenia.
Figs. 4A and 4B illustrates inhibition of RES function by antibodies reactive with soluble OVA (Fig. 4A) or OVA-RBCs (Fig. 4B).
Fig. 5 illustrates that antibodies reactive with soluble OVA or OVA-RBCs both ameliorate immune thrombocytopenia independent of complement activity.
Figs. 6A and 6B illustrate that FcγRIIB expression is required for reversal of immune thrombocytopenia by soluble OVA in the presence of anti-OVA.
Figs. 7A and 7B illustrate that FcγRIIB expression is not required for reversal of immune thrombocytopenia by cell-associated OVA in the presence of anti-OVA.
Figs. 8A and 8B illustrate that antibodies to endogenous soluble antigens ameliorate immune thrombocytopenia.
Fig. 9 illustrates that antibodies to albumin and transferrin require the expression of FcγRIIB to ameliorate immune thrombocytopenia.
Fig. 10A and 10B (not forming part of the present invention) illustrate that antibodies to albumin ameliorate K/BxN serum-induced inflammatory arthritis.

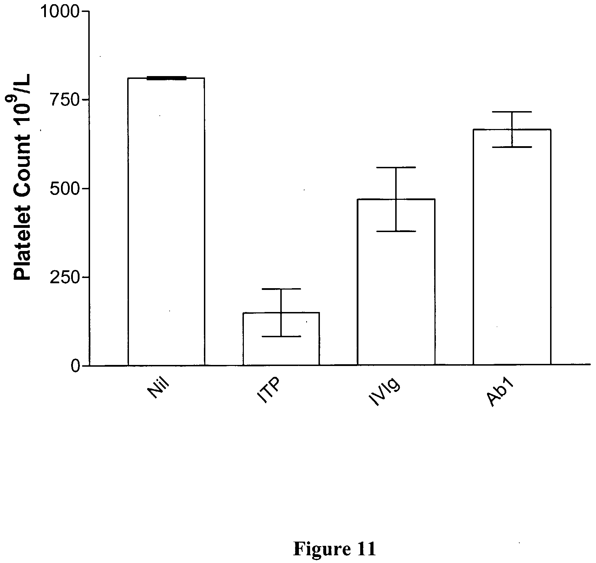

Fig. 11 illustrates IMCP-like effects shown by IVIg and anti-CD44 treatment regimes.
Fig. 12 illustrates IMCP-like effects as shown by IVIg and soluble antigen-specific antibody treatment regimes.
Fig. 13 illustrates IVIg-treated leukocytes showing therapeutic potential in the absence of FCgammaRIIB expression.

### DETAILED DESCRIPTION OF THE PREFERRED EMBODIMENT

In this description by soluble antigen it is meant a molecule that can be incorporated and circulated in the blood stream. Examples of soluble antigens comprise but are not limited to: proteins, glycoproteins, lipids, glycolipids, peptides, nucleic acids, synthetic molecules or complexes or aggregates thereof.

By endogenous antigen it is meant antigens that occur naturally in a mammal and by foreign (or exogenous) antigen it is meant an antigen that is not normally produced by the same individual or species.

According to one embodiment of the present invention, antibodies to soluble antigens were tested for their ability to ameliorate autoimmune diseases. Specifically, the amelioration of thrombocytopenia was tested. To address this question, a murine model of ITP was used to determine whether IgG specific to a soluble prototype antigen could prevent thrombocytopenia. Mice injected with soluble ovalbumin (OVA) or OVA conjugated to RBCs (OVA-RBC) in the presence of anti-OVA, were both significantly protected from immune thrombocytopenia.

Both of these therapeutic regimes functioned independent of complement activity and both regimes also blocked reticuloendothelial function as assessed by clearance rates of fluorescent sensitized syngeneic RBCs. Soluble OVA or anti-OVA alone did not have any direct effect on immune thrombocytopenia in mice. It was found that OVA-RBC + anti-OVA ameliorated immune thrombocytopenia in normal mice and FcγRIIB^{-/-} mice, while soluble OVA + anti-OVA was ineffective in FcγRIIB^{-/-} mice. In addition, IgG specific for murine albumin and specific for transferrin also effectively inhibited ITP. Thus, IgG antibodies directed to soluble antigens can inhibit or reverse immune thrombocytopenia in an FcγRIIB-dependent manner, whereas antibodies directed to a cell-associated antigen function independent of FcγRIIB expression.

### Materials and Methods:

### Reagents:

The monoclonal antibody specific for integrin α_{IIb} (rat IgG_{1κ}, clone MWReg 30) was purchased from BD Pharmigen (Mississauga, ON, Canada). Monoclonal murine anti-OVA (IgG₁, clone OVA-14), rabbit polyclonal anti-OVA, 1-ethyl-3-(3-dimethylamino-propyl) carbodiimide (EDAC), OVA (grade V), and PKH26 red fluorescent cell linker kit were purchased from Sigma (Oakville, ON, Canada). IVIG was Gamimune, 10% from Bayer (Elkhart,. IN). Cobra Venom Factor (CVF), FITC-conjugated F(ab')₂ anti-rabbit IgG, and control rabbit IgG, were purchased from Cedarlane Laboratories Ltd (Hornby, ON, Canada). Rabbit anti-mouse albumin (IgG fraction), and rabbit anti-mouse transferrin (IgG fraction), were purchased from Research Diagnostics (Flanders, NJ). Hemolysin (anti-SRBC rabbit serum) was supplied by Colorado Serum company (Denver, CO). Microdispenser tubes (250 µl) for blood collection were from VWR, (Mississauga, ON)

### Mice:

Female CD1 mice (6-10 wks of age) were purchased from Charles River Laboratories (Montreal, PQ, Canada). C57BL/6 and FcγRIIB^{-/-} (B6; 129S4-*Fcgr2b^{tm1Rav}*/J) mice were purchased from the Jackson Laboratory (Bar Harbor, ME). All mice were housed in the St. Michael's Hospital Research Vivarium.

### Induction and treatment of immune thrombocytopenia:

Mice were rendered thrombocytopenic by intraperitoneal injection of 2 µg anti-platelet (anti-integrin α_{IIb}) antibody in 200 µl phosphate buffered saline (PBS), pH 7.2. ITP was induced by two protocols:

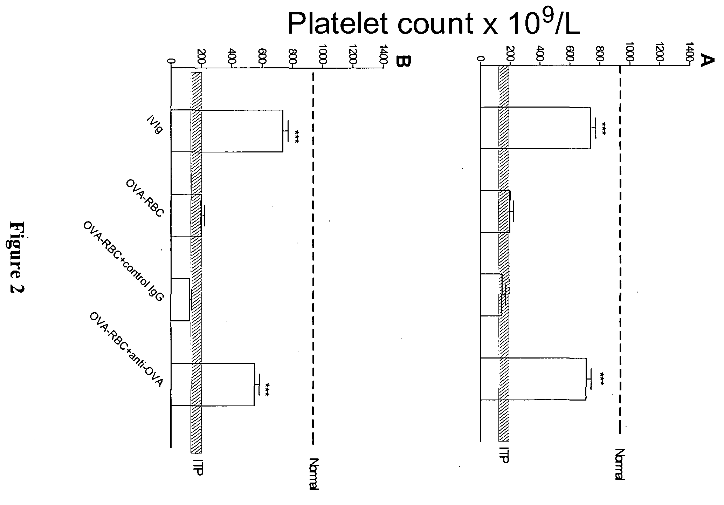

For experiments where the therapeutic intervention preceded the induction of immune thrombocytopenia (e.g. Figs 2, 3, 5), mice were first injected intravenously with the indicated therapeutic preparation (eg OVA-RBC sensitized with anti-OVA IgG), followed at 24 h by a single injection of anti-platelet antibody. Mice were bled for platelet enumeration after a further 24 h.

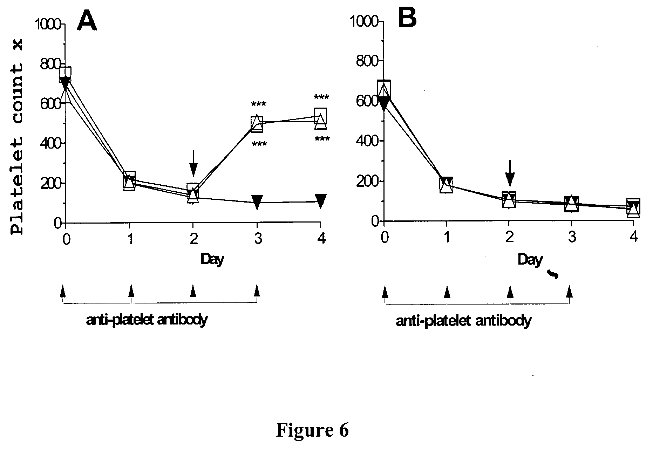

For experiments where the induction of immune thrombocytopenia preceded the therapeutic intervention (e.g. Figs 6-8), mice were injected daily (days 0-3) with anti-platelet antibody and then injected intravenously with the indicated therapeutic preparation (eg OVA-RBC sensitized with anti-OVA IgG) on day 2. Mice were bled daily and platelets counted as described below.

In experiments where we wished to avoid the possibility of the formation of "pre-formed" immune complexes, mice were injected intraperitoneally with soluble OVA only followed 4 hours later by OVA-specific antibody via the intravenous route. Mice injected with anti-albumin or anti-transferrin alone received 1 mg of antibody in a volume of 200 ul on day 2. For all IVIg treatments, mice were injected intraperitoneally with 0.5 ml of 10 % IVIG (roughly equivalent to 2 g/kg body weight). Platelets were counted as follows: Mouse blood (45 µL) was collected via saphenous vein bleeding into microdispenser tubes preloaded with 5 µl of 1% EDTA in PBS. Then, 50 µl of this mouse blood was diluted in 450 µl of 1% EDTA/PBS (1:10) and then further diluted to a final dilution of 1:12,000 in 1% ethylenediaminetetraacetic acid (EDTA)/PBS. Platelets were enumerated on a flow rate-calibrated FACScan flow cytometer (Becton Dickinson, San Jose, CA) using forward scatter (FCS) versus side scatter (SSC) to gate platelets as previously described.

### Preparation of OVA-specific antibody pre-incubated with soluble OVA:

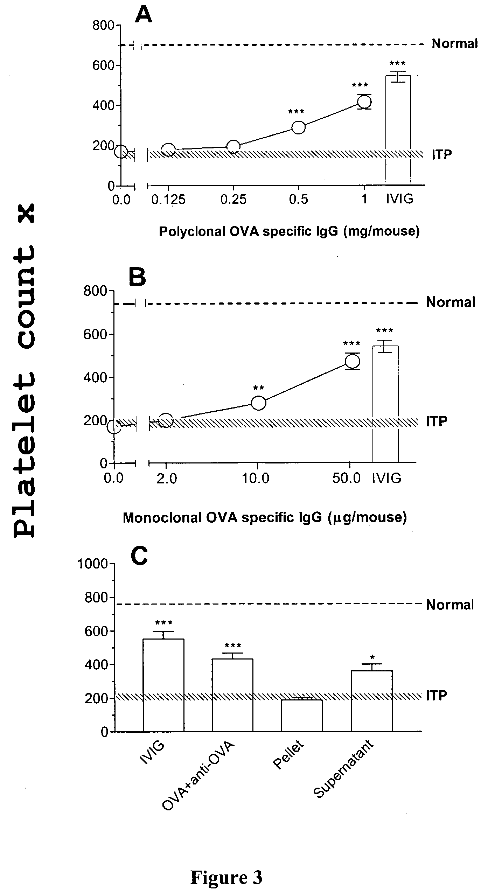

1 mg OVA was dissolved in 300 µl PBS and was incubated with the indicated dose (Fig. 3A, 3B x-axes) of OVA-specific antibody (rabbit polyclonal or mouse monoclonal) for 1 hr at 37°C. The solution was then injected intravenously in a 300µl volume. In separate experiments the OVA and antibody solution was incubated as above for 1 hour at 37°C and macromolecular immune complexes removed by centrifugation at 16,000xg at 4°C for 1 h followed by filtration of the resulting supernatant fluid using a 0.2 µm filter (Filtropur S plus 0.2, Sarstedt, Montreal, PQ). The pellet was resuspended in 300 µl PBS and intravenously injected as above.

### Preparation of OVA-coupled RBCs:

OVA was coupled to RBCs as follows: RBCs were resuspended at 2.5x10⁸/mL in 5 mg/mL OVA in saline and 1.9 mg/mL 1-ethyl-3-(3-dimethylamino-propyl) carbodiimide (EDAC) was added. Following a 1 hr incubation at 4°C, the cells were washed once with a 2 mg/mL solution of OVA in PBS followed by one wash in PBS. To confirm the presence of OVA on RBCs, OVA couple RBCs were incubated with 17 µg/mL rabbit polyclonal anti-OVA, washed, and then incubated with 8 µg/mL FITC conjugated F(ab')₂ anti-rabbit IgG. Cells were washed, resuspended in PBS, and analyzed by flow cytometry.

### Reticuloendothelial system (RES) blockade:

RES blockade was assessed as follows: Whole blood (2 ml, diluted with 1/5 volume 1% EDTA in PBS) from unmanipulated SCID mice was pooled and centrifuged at 2,000 x g for 15 min to obtain 1 ml of packed erythrocytes. These packed erythrocytes were resuspended in 4 ml of PBS and incubated with 8 µg of anti-TER-119 antibody at 22°C for 30 min. The resulting opsonized erythrocytes were then washed twice with PBS and labeled with a fluorescent marker (PKH26 Kit, Sigma, St. Louis MO) according to the manufacturer's directions. Briefly, the opsonized erythrocytes were resuspended in 3 ml of PKH26 'diluent C' and mixed with another 4 ml of 'diluent C' containing 10 µl of the 'PKH26 linker'. After a 5 minute incubation with constant swirling, the mixture was incubated for 5 minutes with an equal volume of PBS containing 1% bovine serum albumin. The erythrocytes were washed 5 times and resuspended in 2 ml PBS. Mice were then injected via the tail vein with 200 µl of these labeled cells. All mice were bled via the tail vein at 3 min, 10 min, 30 min, 120 min, and 960 min post injection and the total number of erythrocytes, as well as the percent of PKH26-fluorescent erythrocytes, were counted by flow cytometry. The percentage of labeled erythrocytes at the 3 min time point was considered to be 100%.

### Complement depletion:

Complement depleted mice were prepared by intraperitoneal injection of 5U of Cobra Venom Factor (CVF) in 200 µl phosphate-buffered saline pH 7.2 followed by a second injection of CVF after 4h. Complement depletion was confirmed by the complement hemolytic activity assay Briefly, sheep RBCs (SRBC) were washed in PBS and resuspended at 1x10⁸/mL. Hemolysin (anti-SRBC rabbit serum) was diluted 1:50 and incubated with these sheep RBCs at 37°C for 30 min, washed in PBS and the cells incubated with a 1:10 dilution of mouse sera from control vs. CVF-treated mice at 37°C for 30 min. The mixture was then diluted with PBS, centrifuged at 1000 xg for 5 min. Complement activity from the sera was assessed as follows: SRBC were resuspended in PBS at 1 x 10⁸/mL. One mL of this was incubated with 1 mL of a 1/50 dilution of anti-SRBC antibody ('Hemolysin', Colorado serum, Denver, CO) and incubated for 30 min at 37°C. Cells were washed in PBS, and adjusted to 1 x 10⁸/mL in PBS. Twenty mL of these cells were added to 20 µl mouse serum from experimental mice in a 96 well flat bottom tissue culture plate for 30 min at 37°C. The plate was then centrifuged at 1,000 x g for 5 min, the supernatant was transferred to a new 96 well plate and the absorbance was read at 540 nm. Calculate percent hemolysis: 100 x (OD₅₄₀sample-OD₅₄₀blank) / (OD₅₄₀max-OD₅₄₀blank). Calculate 50% lysis by plotting the log of serum dilution against log (%lysis/(100-%lysis)).

### Statistical analysis:

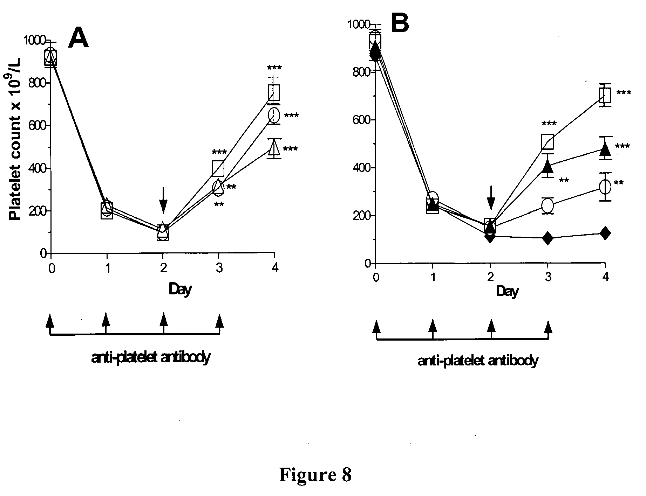

Data was analyzed using the Student's t test, except data in Fig. 8, which was analyzed by one-way ANOVA. The level of significance was set at P< 0.05.

### Results

### Antibodies reactive with a cell-associated antigen can inhibit immune thrombocytopenia:

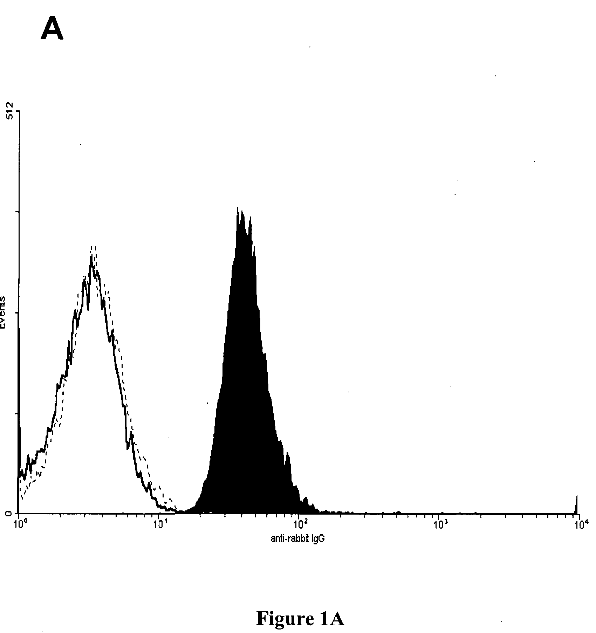

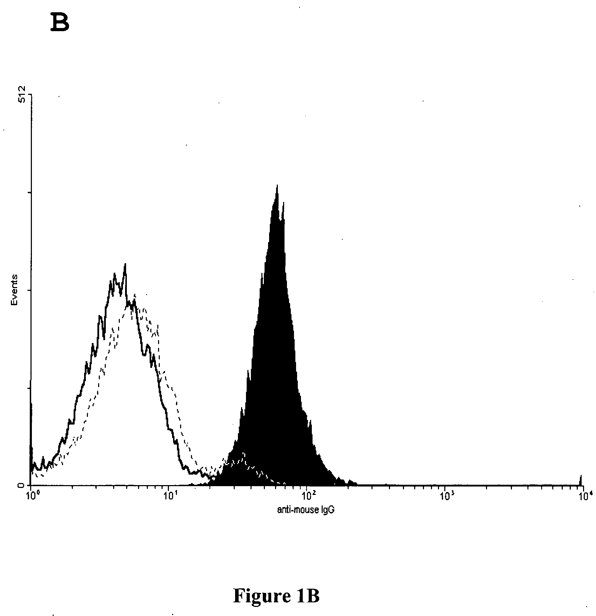

OVA-coupled murine RBCs (OVA-RBC) were assessed for reactivity with mouse (Fig. 1A) and rabbit (Fig. 1B) antibody specific to OVA by flow cytometry to ensure successful coupling of the OVA-RBCs. Figs. 1A and 1B illustrate the association of OVA on the surface of RBCs wherein OVA coupled RBCs are prepared with 1-ethyl-3-(3-dimethylamino-propyl) carbodiimide (EDAC) (Sigma Oakville, ON). OVA was coupled to RBCs as follows: RBCs were resuspended at 2.5x108/mL in 5 mg/mL OVA in saline and 1.9 mg/mL EDAC was added. Following a 1 hr incubation at 4°C, the cells were washed once with a 2 mg/mL solution of OVA in phosphate buffered saline (PBS), pH 7.2 followed by one wash in PBS. The OVA coupled RBCs were stained with rabbit (Fig. 1A) or mouse (Fig. 1B) polyclonal anti-OVA IgG (solid histogram), control rabbit (Fig. 1A) or mouse (Fig. 1B) IgG (solid line), followed by the appropriate FITC conjugated secondary antibody (dashed line, secondary antibody only) and wherein the x axis shows relative fluorescence intensity; y-axis represents cell number.

The monoclonal anti-OVA antibody employed in this study did react with OVA (as assessed by ELISA), but did not react with OVA-RBCs suggesting that the epitope recognized on OVA may be masked upon coupling with RBCs. Thus monoclonal anti-OVA was only used in treatments involving soluble OVA.

CD1 mice were injected intravenously with 1x10⁸ OVA-RBCs pre-incubated with nothing, OVA specific antibodies or an appropriate control IgG, 50 mg IVIg (roughly equivalent to 2g/kg body weight in a 25g mouse), or were left untreated. After 24 hours, all mice received anti-platelet antibody and all mice were bled for platelet enumeration after a further 24 h. Mice that received anti-platelet antibody alone became thrombocytopenic (Figure 2, shaded horizontal bar), compared to unmanipulated control mice (Figure 2, dashed line). Figs. 2A and 2B illustrates inhibition of thrombocytopenia by treating OVA-coupled RBCs with OVA-specific IgG; CD1 mice were pre-injected intravenously with 1x10⁸ OVA-coupled RBCs pre-incubated with either rabbit (A) or mouse (B) OVA-specific polyclonal IgG, control IgG, or anti-OVA antibody, as indicated on the x axis. Mice in the IVIG groups received 50 mg IVIG. All mice (except 'Normal') received anti-platelet antibody one day later. Mice were bled for platelet enumeration after a further 24 h. Normal: The dashed line denotes the mean platelet count of non-injected mice; ITP: The horizontal bar denotes the mean platelet count (± 1 SEM) of mice injected with anti-platelet antibody only. The x-axis indicates treatment; y-axis denotes platelet count; n=9 mice for each data point. *** *P* < 0.001 vs. ITP mice. Data are represented as mean ±SEM.

Mice treated with OVA-RBCs pre-incubated with either 50 µg rabbit polyclonal anti-OVA (Figure 2A, 'OVA-RBC + anti-OVA') or 50 µg murine polyclonal anti-OVA (Figure 2B, 'OVA-RBC + anti-OVA') were significantly protected from the development of immune thrombocytopenia compared with mice receiving OVA-RBCs alone (OVA-RBC) or OVA-RBC + control IgG (OVA-RBC + control IgG). The effectiveness of the IgG coated OVA-RBCs was comparable to that of IVIg (Figure 2A&B).

### Antibodies reactive with a soluble antigen can inhibit immune thrombocytopenia:

CD1 mice were injected intravenously with 1 mg soluble OVA that had been pre-incubated with serial dilutions of the indicated amount of rabbit polyclonal anti-OVA (Figure 3A) or the indicated amount of murine monoclonal anti-OVA antibody (Figure 3B) one day prior to injection of anti-platelet antibody. Both of these therapeutic preparations ameliorated immune thrombocytopenia (polyclonal anti-OVA at dosages of 1.0 or 0.5 mg/mouse, monoclonal at dosages of 50 or 10 ug/mouse).
CD1 mice were pre-injected intravenously with 1 mg OVA pre-incubated with the dose of rabbit polyclonal anti-OVA (A), or mouse monoclonal anti-OVA (B), as indicated on the x axis. Mice in the IVIG groups received 50 mg IVIG. The induction of thrombocytopenia and platelet counting were as in Figure 2. Panel C: the OVA/polyclonal anti-OVA solution was centrifuged and the supernatant fluid filtered using a 0.2 um filter to remove macromolecular immune complexes. The pellet was resuspended in PBS. Mice were injected with the therapeutic preparations indicated on the x axis. The induction of thrombocytopenia, platelet counting, and axis legends are as in Fig 2. The number of mice for data point were n=15 (A, B), n=4 (C). *** *P* < 0.001 vs. ITP mice. Data are represented as mean ± SEM.

It is of interest to note that OVA incubated with 50 ug monoclonal anti-OVA was essentially as successful at inhibiting ITP as was a standard dose of IVIg (Fig 3B). Mice treated with soluble OVA alone (Figure 3A&B, 0.0 mg/mouse) or OVA + control IgG (data not shown) were not significantly protected from the development of immune thrombocytopenia. OVA by itself did not affect the platelet count at any dose tested (0.1 mg, 1 mg, 5 mg and 10 mg, data not shown). Similarly all of the anti-OVA antibodies, in the absence of OVA, did not inhibit immune thrombocytopenia (data not shown).

To determine if the OVA + anti-OVA preparation ameliorated immune thrombocytopenia due to the formation of large macromolecular immune complexes, we subjected the OVA + polyclonal anti-OVA preparation (1mg:1mg) to centrifugation at 16,800 xg for 1 hr. at 4°C and the resulting supernatant was then filtered through a 0.2 uM filter (Filtropur S plus 0.2, Sarstedt, Montreal, PQ). Pretreatment of mice with the filtered supernatant, but not the dissolved pellet(the pellet was dissolved by resuspending the pellet in PBS, pH 7.2, back to the original volume), prior to injection of anti-platelet antibody protected mice from thrombocytopenia (Figure 3C), suggesting that the "active" fraction was soluble and less than 0.2 uM in size.

### Antibodies reactive with soluble and a cell-associated soluble antigen both block RES function:

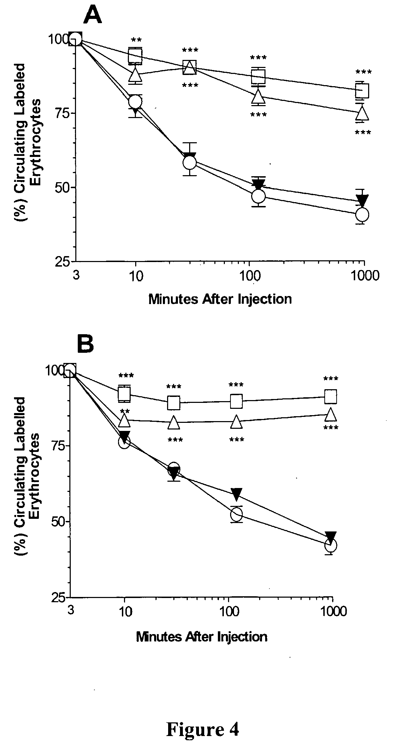

To assess whether the therapeutic regimes under study inhibited RES function, we employed a variation of the classic RES blockade assay, analysing the clearance rate of fluorescently labelled syngeneic RBCs sensitised with a murine RBC-specific antibody (anti-TER-119). Mice were subjected to the indicated therapeutic treatments, and their ability to clear these intravenously injected labelled RBCs over time was analysed (Fig 4). For the soluble antigen studies, mice were injected with nothing, IVIg, OVA-anti-OVA, or control IgG alone for 24 h followed by sensitized fluorescent RBCs (Figure 4A). At the indicated times post sensitized-fluorescent-RBC injection, blood was sampled to assess the RBC clearance rate as a measure of RES function. Only IVIg and OVA-anti-OVA blocked sensitized RBC clearance. Similar results were obtained with murine anti-OVA in combination with soluble OVA (data not shown).

For the cell-associated antigen studies, mice were injected with nothing, IVIg, anti-OVA sensitized OVA-RBCs, or OVA-RBCs alone for 24 h followed by sensitized fluorescent RBCs (Figure 4B). Only IVIg and anti-OVA sensitized OVA-RBCs blocked sensitized-fluorescent-RBC clearance.

In accordance with Figures 4A and 4B, mice were either not pre-treated (O), pre-treated with IVIG (□), pre-treated with 1 mg OVA pre-incubated with 1 mg rabbit anti-OVA (Δ), or pre-treated with 1 mg control IgG + 1 mg OVA (▼), followed 24 hours later by intravenous injection with fluorescently labeled TER-119-opsonized syngeneic RBCs, prepared as follows: Whole blood (2 ml, diluted with 1/5 volume 1% EDTA in PBS) from unmanipulated mice was pooled and centrifuged at 2,000 x g for 15 min to obtain 1 ml of packed erythrocytes. These packed erythrocytes were resuspended in 4 ml of PBS and incubated with 8 µg of anti-TER-119 antibody at 22°C for 30 min. The resulting opsonized erythrocytes were then washed twice with PBS and labeled with a fluorescent marker (PKH26 Kit, Sigma, St. Louis MO) as follows: Briefly, the opsonized erythrocytes were resuspended in 3 ml of PKH26 'diluent C' and mixed with another 4 ml of 'diluent C' containing 10 µl of the 'PKH26 linker'. After a 5 minute incubation with constant swirling, the mixture was incubated for 5 minutes with an equal volume of PBS containing 1% bovine serum albumin. The erythrocytes were washed 5 times and resuspended in 2 ml PBS. Mice were then injected via the tail vein with 200 µl of these labeled cells. All mice were bled via the tail vein at 3 min, 10 min, 30 min, 120 min, and 960 min post injection and the total number of erythrocytes, as well as the percent of PKH26-fluorescent erythrocytes, were counted by flow cytometry. The percentage of labeled erythrocytes at the 3 min time point was considered to be 100%.

Blood samples were taken at the times indicated on the x axis and the percentage of fluorescent RBCs in the circulation assessed by flow cytometry (Fig. 4B), mice were either not pre-treated (○), pre-treated with IVIG (□), pretreated with anti-OVA sensitized OVA-RBCs (Δ), or pre-treated with OVA-RBCs only (▼) followed 24 hours later with intravenous injection of fluorescently labelled TER-119-opsonized autologous RBCs.

### Antibodies reactive with soluble or cell-associated soluble antigen inhibit ITP independent of complement activity:

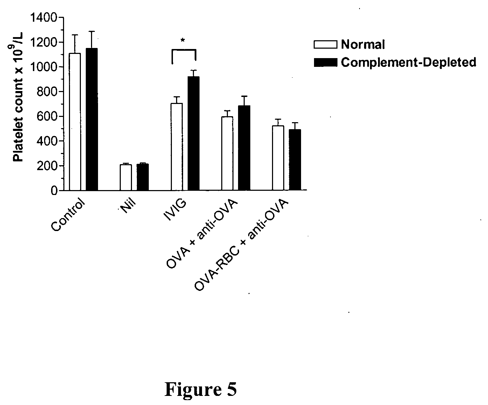

To determine if complement was a contributing factor to the above therapies, mice were depleted of Complement using cobra factor venom (CVF) as described above in [46]._CVF successfully depleted complement from the treated mice as assessed in a hemolytic activity assay on day 3 post CVF-treatment (data not shown). Complement depleted mice developed thrombocytopenia to the same extent as normal mice (Figure 5, column set 2). Complement depleted and normal mice both responded to the protective effects of OVA + anti-OVA and OVA-RBC + anti-OVA (column sets 4 and 5 respectively) to the same extent. However, complement depleted mice responded to IVIg treatment with significantly higher platelet counts compared with normal mice.

As shown in Figure 5, antibodies reactive with soluble OVA or OVA-RBCs both ameliorate immune thrombocytopenia independent of complement activity wherein mice were injected with CVF to deplete complement or were left untreated. After 24 hours, mice were treated with the therapeutic preparations indicated on the x axis, the induction of thrombocytopenia and platelet counting were as in Fig 2, control: mice receiving no therapeutic pre-treatment; Nil: mice treated with anti-platelet antibody only; 'OVA + anti-OVA': mice pre-treated with OVA + anti-OVA, followed 24 hr later by injection of anti-platelet antibody. 'OVA-RBC + anti-OVA': mice pre-treated with OVA-RBC + anti-OVA, followed 24 hr later by injection of anti-platelet antibody.

### FcγRIIB expression is required for protection with antibodies reactive with soluble, but not a cell-associated antigen:

Wild type and FcγRIIB^{-/-} mice were injected daily with anti-platelet antibody (↑) to induce stable thrombocytopenia (Fig 6). Mice were then treated with IVIg, OVA + anti-OVA, or control IgG + OVA on day 2. Treatment of mice with 2 g/kg IVIg as well as OVA + anti-OVA successfully reversed immune thrombocytopenia in wild type (Figure 6A), but neither ameliorated ITP in FcγRIIB^{-/-} mice (Figure 6B). Mice treated with control IgG + OVA displayed no increase in platelet counts.

Figs. 6A and 6B illustrate that FcγRIIB expression is required for reversal of immune thrombocytopenia by soluble OVA in the presence of anti-OVA wherein wild type mice (Fig. 6A) or mice genetically deficient for FcγRIIB (FcγRIIB^{-/-} ) mice (Fig. 6B) were injected with anti-platelet antibody on days 0 through 3 denoted by the arrow (↑), on day 2 (↓) mice were injected intraperitoneally with IVIG (□), or intravenously with OVA + anti-OVA antibody (Δ), or nonspecific IgG + OVA (▼) and mice were bled daily for platelet counts (x10⁹/L).

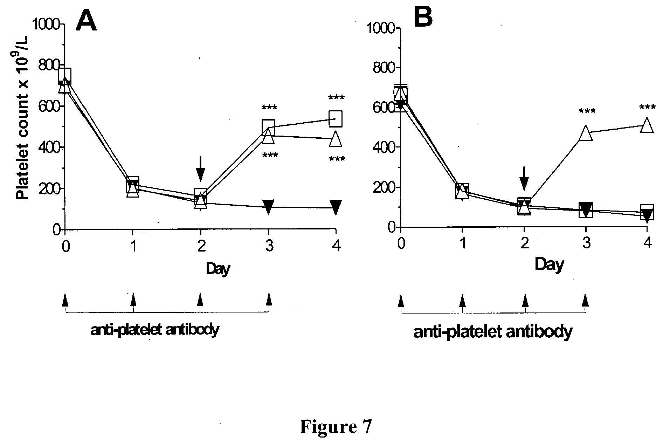

In sharp contrast to the results in Fig 6, ITP was successfully reversed in normal mice (Figure 7A) and FcγRIIB^{-/-} mice (Figure 7B) that were therapeutically treated with OVA-RBCs + anti-OVA. As expected, treatment of mice with OVA-RBCs alone did not increase platelet counts in thrombocytopenic mice. Figs. 7A and 7B illustrate that FcγRIIB expression is not required for reversal of immune thrombocytopenia by cell-associated OVA in the presence of anti-OVA wherein wild type mice (Fig. 7A) or FcγRIIB^{-/-} mice (Fig. 7B) were injected with anti-platelet antibody on days 0 through 3 (↑), on day 2 (↓) mice were injected intraperitoneally with IVIG (□), or intravenously with anti-OVA sensitized OVA-RBCs (Δ), or OVA-RBCs alone.

### Preformation of immune complexes are not necessary for reversal of ITP:

To determine if it is necessary to incubate antigen and antibody before injection to ameliorate the thrombocytopenia in our model, mice were pre-injected with either 1 mg or 10 mg of soluble OVA followed by 1 mg anti-OVA after 4h. Significant reversal of ITP was achieved with OVA specific IgG in mice that were previously treated with either 1mg or 10 mg of OVA (Figure 8A).

To determine if antibody to endogenous soluble antigens can also inhibit immune thrombocytopenia, thrombocytopenic mice were treated with 1 mg polyclonal anti-mouse albumin or 1 mg anti-mouse transferrin antibody on day 2. Both of these antibodies, but not control IgG, significantly ameliorated the immune thrombocytopenia (Figure 8B). As illustrated in Figs. 8A and 8B, antibodies to endogenous soluble antigens ameliorate immune thrombocytopenia wherein (Fig. 8A) mice were treated with IVIG only (□), 10 mg OVA (Δ), or 1 mg OVA (○), followed four hours later by 1 mg OVA-specific IgG (↓) on day 2 and wherein thrombocytopenia and platelet counting were as in Fig 6 and wherein (Fig. 8B) mice were treated with IVIG (□), 1 mg anti-mouse albumin antibody (▲), 1 mg anti-mouse transferrin antibody (○), or control IgG (◆).

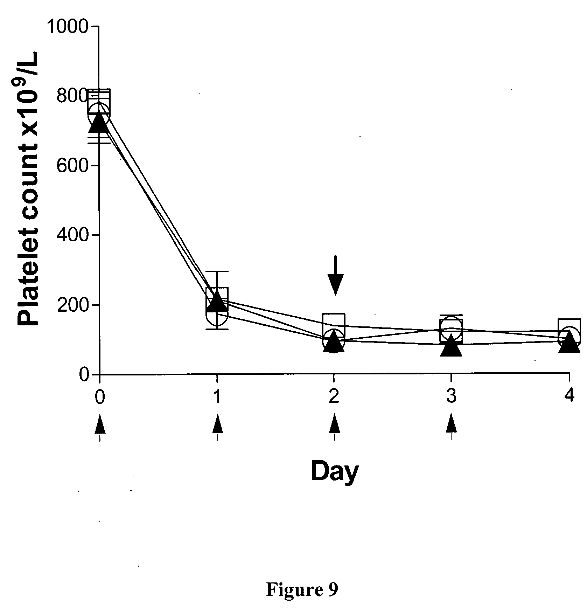

In contrast, anti-mouse albumin and anti-mouse transferrin antibodies failed therapeutically in FcγRUB^{-/-} mice, and did not reverse immune thrombocytopenia (Figure 9). Here, antibodies to albumin and transferrin require the expression of FcγRIIB to ameliorate immune thrombocytopenia. FcγRIIB^{-/-} mice were injected with 2 µg anti-platelet antibody on days 0 through 3 denoted by the arrow (↑). On day 2 (↓) mice were injected intraperitoneally with 50 mg IVIg (□), or intravenously with 1 mg anti-albumin antibody (▲), or 1 mg anti-transferrin antibody (O). Mice were bled daily for platelet counting; n=3 mice for each group. Data are presented as mean ± SEM.

In another embodiment of the invention antibodies to soluble antigens were used to treat or ameliorate inflammatory arthritis.

### Material and methods

### K/BxN Serum-induced arthritis and arthritis scoring:

For induction of arthritis, mice were given a single intraperitoneal injection of 600 µl of diluted serum (diluted to 50% strength with PBS) as previously described by Akilesh et al (Akilesh, S., Petkova, S., Sproule, T.J., Shaffer, D.J., Christianson, G.J., and Roopenian, D. 2004. The MHC class I-like Fc receptor promotes humorally mediated autoimmune disease. J Clin Invest 113:1328-1333.). An additional control group of mice were injected with only PBS instead of K/BxN serum. Ankle width was measured laterally across the joint with a caliper (Samona International, Canada). Arthritis was also clinically scored daily by an independent blinded observer. Each paw was scored as follows: 0, [unaffected], 1 [slight swelling], 2 [moderate swelling], 3 [severe swelling involving the entire paw (foot, digits, ankle)], and the overall score was calculated as the sum of individual scores for each paw as described by de Fougerolles et al (de Fougerolles, A.R., Sprague, A.G., Nickerson-Nutter, C.L., Chi-Rosso, G., Rennert, P.D., Gardner, H., Gotwals, P.J., Lobb, R.R., and Koteliansky, V.E. 2000. Regulation of inflammation by collagen-binding integrins alphalbeta1 and alpha2beta1 in models of hypersensitivity and arthritis. J Clin Invest 105:721-729.). Mice injected with anti-albumin or the IgG control received 1 mg of IgG intravenously in 200 µl PBS four hours prior to the induction of arthritis. Mice injected with IVIg received 50 mg of IVIg by an intraperitoneal injection four hours prior to the induction of arthritis.

### IgG reactive with a soluble antigen can ameliorate arthritis:

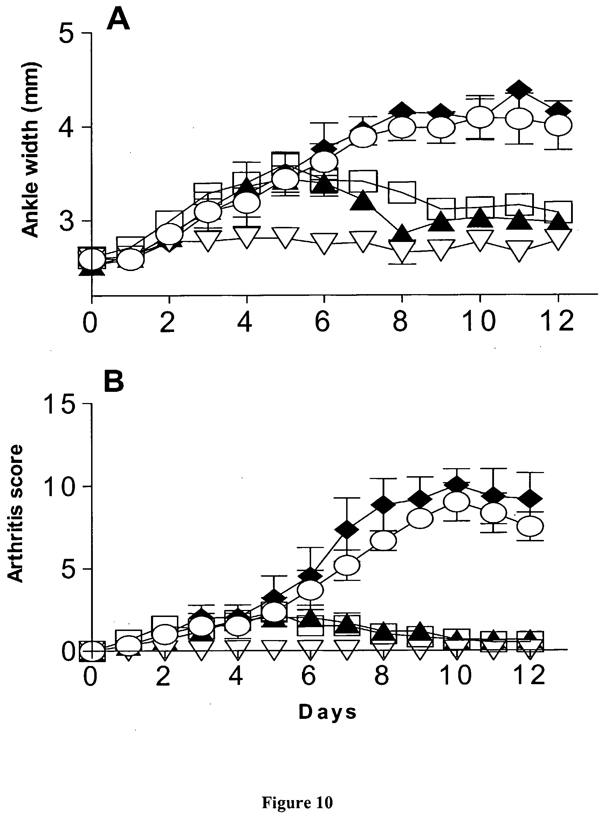

To further evaluate the therapeutic role of antibodies directed to a soluble antigens in the K/BxN serum-induced arthritis model, C57BL/6 mice were injected with 50 mg IVIg, 1 mg anti-albumin, 1 mg non-immune IgG, or nothing 4 hours prior to receiving K/BxN serum. An additional control group of mice were injected with only PBS in place of the K/BxN serum. Mice that received K/BxN serum alone, or K/BxN serum + non-immune IgG, developed joint swelling (Figure 10A and B). As shown in Figures 10A & B, antibodies to albumin ameliorate K/BxN serum-induced inflammatory arthritis. (A) Ankle width and (B) overall arthritis score following K/BxN serum-induced arthritis. C57BL/6 mice were injected on day 0 with K/BxN serum (O), IVIg + K/BxN serum (□), anti-albumin + K/BxN serum (▲), Non-immune IgG + K/BxN serum (◆), or treated with only PBS in place of K/BxN serum (∇). Date represented as the mean ± SEM; n=3 mice for each group.

IVIg and the anti-albumin treatment significantly ameliorated the arthritis as assessed by ankle width measurements as well as by clinical score as compared to mice that received K/BxN serum or K/BxN serum plus treatment with non-immune IgG (Figure 10A and B).

### Mechanism of Action:

Our further investigation has also revealed surprising evidence for the mechanism of action of the treatment regimes as herein disclosed. In particular, we have established that antibody treatment regimes such as IVIg, a monoclonal antibody to CD44 antigen and anti-soluble immune complex antibodies (in the presence of the antigen) work to ameliorate autoimmune disease via an antibody-mediated cellular programming mechanism, otherwise herein referred to as IMCP, of non-B and non-T cell leukocytes. In particular, we show that IVIg, monoclonal antibody to CD44 antigen and anti-soluble immune complex antibodies (in the presence of the antigen) can bind to leukocytes in vitro and upon transfer in vivo, can ameliorate ITP, for example. More specifically, IVIg, a monoclonal antibody to the CD44 antigen, and anti-soluble immune complex antibodies (in the presence of the antigen) ameliorate autoimmune disease by interacting with a non-B cell non-T cell leukocyte which then, upon transfer to a host with an autoimmune disease, ameliorates disease activity. We have found that the leukocyte which mediates these clinical effects co-purifies with cells, including a subset of intestinal epithelial lymphocytes and a subset of activated T-cells, expressing the CD11c cell surface antigen, a surface marker expressed on most dendritic cells [data not shown]. Thus, a novel mechanism of action for IVIg and IVIg-like treatment regimes for autoimmune disease is herein provided.

Furthermore, a common linking factor is established in that the expression of FCgamma RIIB inhibitory receptor on cells is shown in the treatment regimes for anti-CD44 and antibodies directed to a soluble antigens, as has been previously established for IVIg. Thus, providing evidence that a common mode of action is the basis for the treatment regimes of the present invention. Having established a common mechanism of action with IVIg, anti-CD44 antibody, we believe that an antibody for a soluble antigen, in accordance with the present invention, will have a similar therapeutic effect as IVIg or anti-CD44 antibody, in the treatment and/or amelioration of a plurality of autoimmune diseases. Accordingly, the embodiments of the present invention may be extended to provide beneficial treatment regimes for the prevention and/or treatment of other autoimmune diseases.

### Materials and Methods

### Mice:

CD1 mice (female 6-10 wk of age) and severe combined immune deficient (SCID) virgin mice (female 6 to 8 weeks of age) were purchased from Charles River Laboratories (Montreal, PQ, Canada). C57BL/6, BALB/c, and FcγRIIB^{-/-} mice were (female 8 to 12 weeks of age) were from the Jackson Laboratory (Bar Harbor, ME).

### Reagents:

The monoclonal antibody specific for integrin αIIb (rat IgG_{1κ}, clone MWReg 30) was purchased from BD Pharmingen (Mississauga, ON). Bovine serum albumin (BSA) was purchased from Sigma (Oakville, ON, Canada). The IVIg (Gamimune N, 10%) was from Bayer (Elkhart, IN). To neutralize the pH of the IVIg (in some experiments), both IVIg and BSA were dialysed against phosphate buffered saline (PBS) (pH 7.2) in 1:200 ratio for 18 hours at 4°C using 12-14 kDa cutoff dialysis tubing (Spectrum Laboratories Inc, Rancho Dominguez, CA) under sterile conditions. Microdispenser tubes (250 µL) for blood collection were from VWR. Complete RPMI-1640 was RPMI-1640 medium (Sigma, Oakville, ON, Canada) supplemented with 10% heat-inactivated fetal calf serum, 80 µg/ml streptomycin sulphate, 0.2 µg/ml amphotericin B, 80 U/ml penicillin G and1.6 mM L-glutamine.

### IVIg-Mediated Cellular Programming (IMCP):

### Preparation of IMCP blood:

Blood (400 µl, or as otherwise indicated) was collected in sterile PBS containing 1% EDTA (PBS/EDTA), washed and the cell pellets resuspended in 25 mg/ml of IVIg or BSA in PBS/EDTA. After incubation for 20 min (or as otherwise indicated) at 37°C in a shaking incubator, the cells were washed 2x in Ca⁺⁺ and Mg⁺⁺ free PBS, resuspended in saline and immediately injected back into the original mice. For preparation of WBC-reduced blood cells, the collected blood was first centrifuged at 900 xg for 5 min at 4°C, the plasma and buffy coat fractions were discarded. The cell pellets were washed 3x in PBS and resuspended in 25 mg/ml of IVIg or BSA as described above.

### Preparation of IMCP splenic cells:

Spleens from normal mice were removed, mechanically disrupted in 5 ml of complete RPMI-1640 medium, and then filtered through 70-µm nylon mesh strainer. Erythrocytes were lysed using 0.15 M NH4Cl, 10 mM KHC03, 0.1 mM Na2 EDTA (ACK) lysis buffer and washed 2x in RPMI-1640. The cells (1.4x106/ml) were incubated with 18 mg/ml dialyzed IVIg (IMCP) or BSA (IMCP-control), or the indicated concentration (x/ml) of anti-CD44 (Antibody clone KM-114 or IM7), or with 1 mg of ovalbumin that was pretreated with 50 ug monoclonal anti-ovalbumin (Clone OVA-14, antibody subclass IgG1, From Sigma), or 1 mg of ovalbumin that was pretreated with 50 ug normal mouse IgG (Catalogue # 10400, from Caltag) for 30 min at 370C in RPMI-1640. The cells were then washed 2x in RPMI-1640, resuspended to 5x106/ml and injected (200 µl) into the tail vein of recipient mice.

### Fixation:

Pre-fixed cells: splenic leukocytes (2.5 x106/ml) were fixed in 1% paraformaldehyde in PBS for 10 minutes, washed 2x in PBS and then incubated with IVIg or BSA for 30 min as described above.

Post-fixed cells: splenic leukocytes were first incubated with IVIg or BSA for 30 min as described above, washed 2x in PBS and then fixed in 1% paraformaldehyde in PBS. The cells were then washed 2x in PBS, resuspended at 5x106/ml and injected (200 µl) into the tail vein of recipient mice.

### Radiation:

Splenic leukocytes (5x106/ml) were irradiated (2500 rads) using cell irradiator ( γ source, Cs-137) and then incubated with IVIg or BSA as described above.

### Induction and treatment of ITP:

For the administration of IVIg, BSA, or IMPC-cells, mice were first injected intraperitoneally with 50 mg of IVIg, BSA (∼equivalent to 2 g/kg body weight), IMPC cells, or control-IMCP cells. After 24 hrs, mice were rendered thrombocytopenic by the intraperitoneal injection of 2 µg anti-CD41 (anti-integrin αIIb) antibody in 200 µL PBS. Twenty-four hours later, mice were bled by the saphenous vein and the platelets were counted on a flow rate-calibrated FACScan flow cytometer (Becton Dickinson) as previously described in detail (Br. J. Haematol. 115:679-686, 2001; Blood.101: 708-3713, 2003).

### T cell purification:

T cells were purified from spleens by magnetic separation using a T cell negative selection kit (StemCell Technologies, Vancouver, BC) according to manufacturer's instructions. Briefly, splenocytes were prepared in Ca++ and Mg++ free PBS containing 2% heat-inactivated fetal calf serum and 5% normal rat serum at 108 nucleated cells/mL. Splenocytes were then incubated with T cell negative selection cocktail (containing antibodies to CD11b, CD45R, Ly-6G(Gr-1), TER 119) at 20 µl/mL, followed by biotin selection cocktail at 100 µl/mL, and magnetic nanoparticles at 100 µl/mL. All incubations were done for 15 min at 4oC. The recovered cells were stained with anti-CD3-FITC (10 µg/mL) and anti-CD19-PE (4 µg/mL) for 30 min at 4oC, washed, and analyzed by a FACScan flow cytometer. The recovered cells were routinely >90% CD3+ and <1% CD19+.

### B cell purification:

B cells were purified from the spleen by magnetic separation using a B cell negative selection kit (StemCell Technologies, Vancouver, BC) according to manufacturer's instructions. Briefly, splenocytes were prepared in Ca++ and Mg++ free PBS containing 2% heat-inactivated fetal calf serum and 5% normal rat serum at 108 nucleated cells/mL. Splenocytes were then incubated with mouse FcR blocker (anti-CD16/32) at 10 µl/mL, B cell negative selection cocktail (containing antibodies to CD4, CD8, CD11b, Ly-6G(Gr-1), TER 119) at 20 µl/mL, followed by biotin selection cocktail at 100 µl/mL, and magnetic nanoparticles at 100 µl/mL. All incubations were done for 15 min at 4oC. The recovered cells were stained with anti-CD3-FITC (10 µg/mL) and anti-CD19-PE (4 µg/mL) for 30 min at 4oC, washed, and analyzed by FACScan flow cytometer. The recovered cells were routinely >80% CD19+ and 10 % CD3+.

### Results

We found that leukocytes can be treated with IVIg in vitro, washed free of unbound IVIg, and when as little as 106 of these cells are injected into a mouse, an IVIg-like effect is observed (ie. rapid reversal of the autoimmune disease symptom, in ITP, thrombocytopenia). This effect is specifically observed with blood or splenic leukocytes, but not red blood cells. The leukocytes must also be biologically active (ie γ irradiated or paraformaldehyde fixed leukocytes do not work) indicating that simple passive transfer of the IVIg is not the mode of action. B and T cells are not required for this clinical effect of IVIg. Thus, we have strong experimental evidence that the antibody-based treatment regimes of the present invention, induce a priming event in innate leukocytes which endows leukocytes with the ability to ameliorate or inhibit autoimmune disease, specifically in ITP, thrombocytopenia, or in inflammatory arthritis, joint inflammation. We call this effect "IVIg-mediated cellular programming" (IMCP). This term is intended to more broadly refer to an antibody-mediated cellular programming effect, however for simplicity reference is made to the IVIg example, and hence IMCP is used throughout without prejudice. It is not intended to restrict the effect to only IVIg treatment regimes.

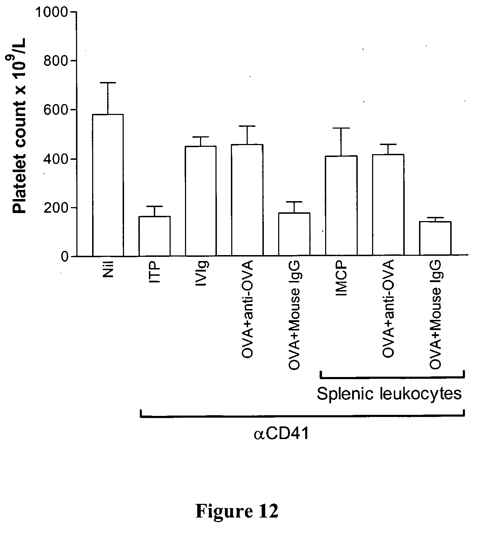

A monoclonal antibody (anti-CD44) is also demonstrated to inhibit immune thrombocytopenia by the same mechanism (ie. an IMCP-like effect in Figure 12. Here, anti-CD44 + leukocytes were incubated for 30 min, unbound anti-CD44 was washed off, leukocytes were then injected into ITP mice, and an amelioration of thrombocytopenia resulted. Mice in the first column (Nil) were uninjected. Mice in the second column (ITP) were treated with anti-platelet antibody (αCD41) only. On Day 1, mice in the third and fourth column (IMCP) were injected intravenously with splenic leukocytes (106/mouse) that went through the IMCP process with IVIg or anti-CD44 for 30 min. On Day 2 mice in columns (second to fourth) were injected with 2 µg anti-platelet antibody. On Day 3, all mice were bled for platelet enumeration as described (Blood 105:1546-1548, 2005).

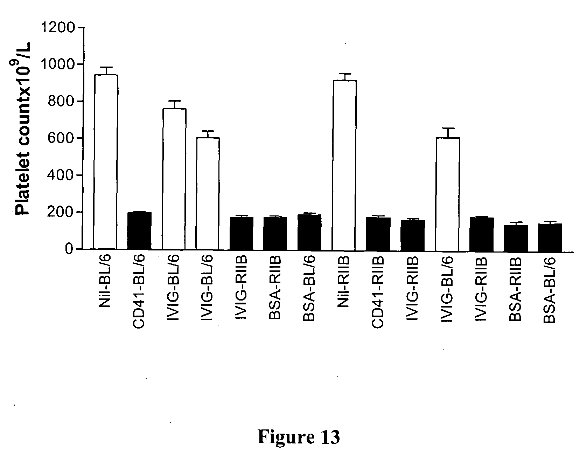

Figure 13 illustrates an antibody-mediated cellular programming effect, herein referred to as IMCP, as mentioned above, at work in splenic leukocytes incubated with monoclonal anti-OVA, thus establishing a basis for the mode of action of the treatment regimes of the present invention. As illustrated, anti-ovalbumin + ovalbumin + leukocytes are incubated for 30 min, unbound anti-ovalbumin and ovalbumin are washed off, and leukocytes are injected into ITP mice to provide ameliorating effect against thrombocytopenia in vivo. According to Figure 13, mice in the first column (Nil) were uninjected. Mice in the second column (ITP) were treated with anti-platelet antibody (αCD41) only. On Day 1, mice in the third column (IVIg) were injected with 50 mg/ml of dialyzed IVIg. Mice in the fourth column were injected (i.v.) with 1 mg OVA that had been pre-incubated with 50 µg of monoclonal anti-OVA (IgG1, clone OVA-14 Sigma). Mice in the fifth column were treated as in fourth column except with control mouse IgG (mouse IgG, Cat# 10400, Caltag) in place of monoclonal anti-OVA. Mice in the sixth column (IMCP) were injected intravenously with splenic leukocytes (106/mouse) that went through the IMCP process with dialyzed IVIg for 30 min. Mice in the seventh column were treated with splenic leukocytes (106/mouse) that went through IMCP process with 1 mg OVA that had been pre-incubated with 50 µg of monoclonal anti-OVA for 30 min. Mice in the eighth column were treated as in seventh column except with control mouse IgG in place of monoclonal-anti-OVA. On Day 2, mice in columns (second to eighth) were injected with 2 µg anti-platelet antibody. On Day 3, all mice were bled for platelet enumeration as described (Blood 102:558-560, 2003).

IVIg, anti-CD44 (KM-114), and antibody to soluble antigens (in the presence of the soluble antigen) cannot ameliorate thrombocytopenia in mice which are genetically deficient in the inhibitory Fcγ receptor (FcγRIIB) Interestingly, however, we show here that these same antibodies can, all ameliorate thrombocytopenia when they are pre-incubated with leukocytes isolated from mice that are genetically deficient in FcγRIIB (FcγRIIB^{-/-}) and the FcγRIIB^{-/-} leukocytes are injected into wild type mice. Thus, the IMCP effect as herein reported can work where leukocytes do not express an FcgammaRIIB receptor. Although, FcgammaRIIB receptor expression was required in the recipient in order to achieve IMCP. In the reverse of this experiment (where the leukocytes are from FcγRIIB^{+/+} mice and the recipient mice are FcγRIIB^{-/-}), again, IVIg, anti-CD44, and anti-soluble antigen (+ the antigen) all cannot ameliorate the thrombocytopenia (Figure 14). As shown in Figure 14, mice in the 1^{st} column (Nil-BL/6) are uninjected C57BL/6 mice. Mice in the 2^{nd} column (CD41-BL/6) were C57BL/6 mice treated with anti-platelet antibody (αCD41) only. Mice in the 8^{th} column (Nil-RIIB) were uninjected FcγRIIB^{-/-} mice. Mice in the 9^{th} column (CD41-RIIB) were FcγRIIB^{-/-} mice treated with anti-platelet antibody (αCD41) only. On Day 1, mice in the 3^{rd} column (IVIG-BL/6) were injected with 50 mg/ml IVIg. Mice in the fourth column (IVIG-BL/6) were C57BL/6 mice injected intravenously with splenic leukocytes (10⁶/mouse) from C57BL/6 mice that went through the IMCP process with IVIg for 30 min. Mice in the 5^{th} column (IVIG-RIIB) were FcγRIIB^{-/-} mice injected intravenously with splenic leukocytes (10⁶/mouse) from C57BL/6 mice that went through the IMCP process with IVIg for 30 min. Mice in the 6^{th} column (BSA-RIIB) were FcγRIIB^{-/-} mice injected intravenously with splenic leukocytes (10⁶/mouse) from C57BL/6 mice that went through the IMCP process with BSA for 30 min. Mice in the 7^{th} column (BSA-BL/6) were C57BL/6 mice injected intravenously with splenic leukocytes (10⁶/mouse) from C57BL/6 mice that went through the IMCP process with BSA for 30 min. Mice in the 10^{th} column (IVIG-RIIB) were injected with 50 mg/ml IVIg. Mice in the 11^{th} column (IVIG-BL/6) were C57BL/6 mice injected intravenously with splenic leukocytes (10⁶/mouse) from FcγRIIB^{-/-} mice that went through the IMCP process with IVIg for 30 min. Mice in the 12^{th} column (IVIG-RIIB) were FcγRIIB^{-/-} mice injected intravenously with splenic leukocytes (10⁶/mouse) from FcγRIIB^{-/-} mice that went through the IMCP process with IVIg for 30 min. Mice in the 13^{th} column (BSA-RIIB) were FcγRIIB^{-/-} mice injected intravenously with splenic leukocytes (10⁶/mouse) from FcγRIIB^{-/-} mice that went through the IMCP process with BSA for 30 min. Mice in the 14^{th} column (BSA-RIIB) were C57BL/6 mice injected intravenously with splenic leukocytes (10⁶/mouse) from FcγRIIB^{-/-} mice that went through the IMCP process with BSA for 30 min. On Day 2, mice in columns (2^{nd} to 7^{th} and 9^{th} to 14^{th}, inclusive) were injected with 2 µg anti-platelet antibody. On Day 3, all mice were bled for platelet enumeration as described in Blood 102:558-560, 2003 with the exception that mice were bled by the saphenous vein in accordance with this embodiment of the present invention.

We therefore conclude that IVIg, anti-CD44, and anti-soluble antigen (in the presence of the antigen) do not function by binding to the FcγRIIB on the leukocyte but do all function by a highly related mechanism, which we refer to as an IVIg-mediated cellular programming mechanism, or IMCP. Furthermore, the cellular programming mechanism (IMCP) of the present invention establishes an underlying mode of action for antibody-based treatment regimes of the present invention that appears to be more accurate than the previously reported RES blockade mechanism.

### DISCUSSION

We have observed that antibodies to soluble antigens ameliorated both murine ITP as well as arthritis. Since the immunological mechanisms involved in both of these diseases is very different, i.e. phagocytosis of opsonized platelets in the spleen vs. joint destruction, our data demonstrate that the therapeutic effects of the anti-soluble-antigen regime work to ameliorate autoimmune disease, in general. In addition to the effectiveness of this treatment regime in both ITP and arthritis treatment, we have also established an underlying mechanism of action for the anti-soluble-antigen regime that is common to that of IVIg (the standard therapy for a multitude of autoimmune diseases) and anti-CD44 antibody. That is, an antibody-mediated cellular programming effect, as illustrated with pre-incubated leukocytes. Thus, further supporting the potential of an anti-soluble-antigen treatment regime of the present invention in the treatment of a plurality of autoimmune diseases.

The above described antibodies and antibody-antigen and antibody-antigen-cell complexes can be incorporated in pharmaceutical compositions to be injected in the mammal. Such compositions may also comprise a pharmaceutically acceptable carrier as would be known in the art.

The compositions can be injected in the mammal by several routes of administration comprising intravenously, interperitoneally, intradermally, intramuscularly, subcutaneously, orally or rectally.

It will be appreciated by persons skilled in the art that other antigens and antibodies could also be used according to the above described method to achieve similar results. It will also be appreciated that the method and composition could be applied to mammals, other than mice and rabbits, such as humans.

The embodiment(s) of the invention described above is(are) intended to be exemplary only. The scope of the invention is therefore intended to be limited solely by the scope of the appended claims.

## Claims

1. A pharmaceutical composition for use in treating an immune thrombocytopenia in a mammal characterized as comprising (i) antibody-antigen complexes obtained by incubating at least one IgG antibody specific for ovalbumin together with ovalbumin and (ii) a pharmaceutical acceptable carrier.

2. Use of at least one IgG antibody specific for ovalbumin for the manufacture of a medicament for the treatment of an immune thrombocytopenia, **characterized in that** ovalbumin and said IgG antibody are incubated together prior to the administration to said mammal to form antibody-antigen or antibody-antigen-blood cell complexes for manufacturing the medicament.

3. The use according to claim 2, **characterized in that** the therapeutic amount of said at least one IgG antibody is from 0.1 µg to 1 g per kg of body weight per day.

## Patentansprüche

1. Pharmazeutische Zusammensetzung zur Verwendung bei der Behandlung einer Immunthrombozytopenie in einem Säuger, **dadurch gekennzeichnet, dass** sie (i) Antikörper-Antigen-Komplexe, die durch Inkubieren von wenigstens einem IgG-Antikörper, der für Ovalbumin spezifisch ist, zusammen mit Ovalbumin gewonnen werden, und (ii) einen pharmazeutisch verträglichen Träger umfasst.

2. Verwendung von wenigstens einem IgG-Antikörper, der für Ovalbumin spezifisch ist, zur Herstellung eines Medikaments für die Behandlung einer Immunthrombozytopenie, **dadurch gekennzeichnet, dass** das Ovalbumin und der IgG-Antikörper vor der Verabreichung an den Säuger gemeinsam inkubiert werden, um Antikörper-Antigen- oder Antikörper-Antigen-Blutzellen-Komplexe zur Herstellung des Medikaments zu bilden.

3. Verwendung nach Anspruch 2, **dadurch gekennzeichnet, dass** die therapeutische Menge des wenigstens einen IgG-Antikörpers 0,1 µg bis 1 g pro kg Körpergewicht pro Tag beträgt.

## Revendications

1. Composition pharmaceutique pour une utilisation dans le traitement d'une thrombocytopénie immunitaire chez un mammifère, **caractérisée en ce qu'**elle comprend (i) des complexes anticorps-antigène obtenus par incubation d'au moins un anticorps IgG spécifique pour l'ovalbumine conjointement avec de l'ovalbumine et (ii) un support pharmaceutique acceptable.

2. Utilisation d'au moins un anticorps IgG spécifique pour l'ovalbumine pour la fabrication d'un médicament pour le traitement d'une thrombocytopénie immunitaire, **caractérisée en ce que** l'ovalbumine et ledit anticorps IgG, sont incubés conjointement avant l'administration audit mammifère pour former des complexes anticorps-antigène ou anticorps-antigène-cellule sanguine pour la fabrication du médicament.

3. Utilisation selon la revendication 2, **caractérisée en ce que** la quantité thérapeutique dudit au moins un anticorps IgG est de 0,1 µg à 1 g par kg de poids corporel par jour.
